# EUROPEAN PATENT APPLICATION

(11) **EP 3 632 506 A1**
(43) Date of publication of application: **08.04.2020**
(21) Application number: 17912261.9
(22) Date of filing: 29.05.2017
(51) Int. Cl.: A61N 1/06, A61B 18/14, A61N 1/32

(54) **ACTIVE INTRACAVITARY ACCESSORY**

(71) Applicant: Sanchez Jaime, Maria del Pilar, 08970 Barcelona (ES)
(72) Inventor: Sanchez Jaime, Maria del Pilar, 08970 Barcelona (ES)
(74) Representative: Isern Patentes y Marcas S.L.
(86) International application number: PCT/ES2017/070364
(87) International publication number: WO 2018/220240

(57) **Abstract**

The invention relates to an active accessory for applying diathermy by radio frequency, applied by contact electrodes in the body cavities, which comprises: a grip (1); an intermediate stem (2); a distal area (3); at least one insulated active electrode (31), arranged in the distal area (3); and a cable (4) for connecting to a radio frequency current generator. The grip (1) has a shaped member (11) that can be felt by touch when holding the grip (1), arranged conveniently to provide a reference indicating the position of the electrode (31), each electrode of the accessory being made up of a metal electrode insulated by a ceramic, plastic or resin coating, with a thickness of 20 to 500 micrometres. In the embodiment, the intermediate stem (2) has an electrode (21) for supplying radio frequency current.

## Description

### Object of the invention

The object of the present invention is an active intracavitary accessory for the application of radiofrequency diathermy applied by contact electrodes in the body cavities, and in particular in the mouth, rectum or vagina.

### Field of application of the invention

This invention is applicable in different medical specialties and especially, but not limited to urology; gynecology, physiotherapy and dentistry.

### Backgrounds of the invention

Various devices for applying heat to different parts of the body are currently known.

Specifically, US 3 848 607 A discloses a therapeutic device for the oral area comprising an applicator for supplying heat through the palate of the mouth. This device comprises an electric heater, which heats a fluid, and a pump that circulates the heated fluid through the manual applicator in the mouth. The applicator includes a handle connected at one end with a flexible tube and, on the other, with a pad of flexible material through a releasable coupling, through which the heated fluid is circulated allowing the transfer of heat to the area to be treated.

The portion of the handle that joins the pad is of relatively rigid material so that it allows the user to gently press the pad against the palate. This portion forms an angle with the handle to facilitate the application.

US 2012322022 A1 discloses a device for testing the health and dental sensitivity of a patient comprising an member by which a stimulus, such as cold or heat, and a control regulating the duration and magnitude of the stimulus is released. The device comprises a body with an interface that allows the user to select the commands, a control, a connection for the tip, a power supply and a power input, as well as an electronic display that shows the information. It also comprises a rubber tip for the stimulus to be applied, for example a heat conductive rubber tip. At the tip there may be a sensor that determines the amount of the stimulus that is applied to the patient's tooth and/or the stimulus that returns to the tip.

US 6322583 B1 discloses a device for treating gum pathologies such as polyps or gingivae by massage and heat. The equipment has a system to monitor the temperature and a mechanism for controlling temperature. It comprises a handle that houses a cavity and that joins a tubular member, means for producing energy and an on/off button on the handle. furthermore, it has a temperature sensor that is preferably located near the tip of the tubular member, which can take the form of a "U" to better adapt to the area of the gum to be treated. If the temperature rises above the programmed limit, the temperature control system cuts off the current.

The aforementioned background has adequate features for the application of heat, specifically in the area of the mouth, and does not have features especially indicated for the application of radiofrequency diathermy by means of contact electrodes to body cavities that have different morphologies and that require different types of applications, for example in regard to pressure control or access to different internal areas of said cavities

In the national patent application P201530490, of the same applicant of the present invention an intra-oral member is described for energy application treatments in mandibular bone, gums or other tissues inside the mouth, in particular physiotherapeutic treatments based on application of heat energy, of thermal or athermal effect more or less deep, intended for the molecular stimulation by means of radiofrequency currents, heat radiation or other heat energies generated by an equipment generating said power to which the member is connected.

This intrabuccal member comprises a main body joined, at one end, to a cable for connecting to generating equipment which generates energy and, at the other end, to a shaft with a head at the distal end thereof, wherein it incorporates the energy application means.

In this intra-oral member, the energy application means, constituted by one or more electrodes, are located exclusively at the distal end of the shaft or rod, which makes it difficult to apply energy in large areas of the intracorporeal cavities.

Therefore, the technical problem that arises in the development of an active accessory for the specific application of radiofrequency diathermy, by means of contact electrodes, in various body cavities, which provides a greater area of application of the radiofrequency current, maintaining a reduced contour that facilitates the application inside the body cavities and that presents an improved insulation for the application of radiofrequency current.

### Description of the invention

The intracavitary active accessory of the invention, usable in the application of radiofrequency diathermy by means of contact electrodes in the body cavities, has several versions, all with a perfectly defined common structure, but with the necessary adaptations to optimize its use depending on the areas of the human body that are the target of treatment.

This accessory has a known general structure, with three different parts that make up: a grip, a rod, cylindrical or conical-shaped trunk that extends from an anterior end of the grip and a distal area with an electrode for the application of radio frequency current.

Said accessory has a connection cable to a radiofrequency current generating equipment. According to the invention, the grip includes a shaped member arranged in a certain peripheral area of the grip and which allows the lateral orientation of the electrode of the distal area to be determined by touch during the application of radiofrequency current in a body cavity, and therefore with the electrode in a non-visible position.

In this invention it is provided that the intermediate stem is made of insulating material, so that the device performs the localized application of radiofrequency exclusively by means of the electrode located in the distal zone; or that said intermediate stem incorporates an electrode for application of radio-frequency current in an area of greater surface area, in combination with the electrode of the distal zone.

In the different embodiments of the active device, the electrodes have a construction optimized for applying radiofrequency to the human body by capacitive coupling. Said electrodes consist of a metal electrode isolated by a ceramic, plastic or resin coating, with a thickness between 20 and 500 micrometers, and have a shape adapted to the area to be treated with radiofrequency current. These coating thicknesses ensure correct electrode insulation, without a significant loss of the ability to apply the radiofrequency current.

The incorporation of this ceramic, plastic or resin coating of the mentioned thickness provides adequate electrode insulation and enables the effective and safe application of the radiofrequency current to the body.

Said electrodes incorporate a temperature sensor that allows the temperature measurement of the treated area.

To ensure hygiene and prevent infections, intracavitary members (intermediate stem and distal area) are used with disposable protective covers, type covers probes, of biocompatible material.

In one embodiment of the invention, the accessory preferably comprises at its distal zone, at least one temperature sensor connected to the programmable radio frequency generating equipment. This feature allows the device to self-regulate the temperature of the accessory if it is programmed at a certain temperature.

This accessory is intelligent and incorporates a fingerprint for identification individually by the programmable radio frequency generator equipment.

These and other features of the invention will be more readily understood in view of the exemplary embodiments shown in the attached figures, which are described below.

### Description of the drawings

As a complement to the present description, and for the purpose of helping to make the features of the invention more readily understandable, said description is accompanied by a set of drawings which, by way of illustration and not limitation, represent the following:
Figure 1 shows a perspective view of an embodiment of the intracavitary active accessory, for the application of radiofrequency diathermy by means of contact electrodes in the body cavities, preferably in the vagina and rectum, provided in this case with a distal area with angled electrode and a cylindrical stem with electrode
Figure 2 shows an elevation view of a variant embodiment of the active intracavitary accessory of the previous figure with the straight distal area.
Figure 3 shows a variant embodiment of the active intracavitary accessory according to the invention for the application of radiofrequency current in the temporomandibular zone, provided in this case with an insulating stem and a hemispherical distal zone with an electrode.
Figure 4 shows a variant embodiment of the active intracavitary accessory according to the invention for applying radiofrequency current in soft tissues of the mouth, provided with a distal area, spherical angle electrode and insulating rod.
Figure 5 shows a variant embodiment of the active intracavitary accessory according to the invention for applying radiofrequency current in the gum area, provided with an insulating stem covered with a soft material and with a distal area incorporating an electrode with reinforced rear part in insulating material.
Figure 6 shows an elevational view of an embodiment of a sheath filled with electric conductive gel, sectioned by a vertical plane and arranged in use position on the front end of the intracavitary active accessory of Figure 5.

### Preferred embodiment of the invention

In the exemplary embodiments shown the attached figures the intracavitary active accessory of the invention comprises three different portions that comprise: a grip (1), an intermediate stem (2) of cylindrical or frustoconical shape extending from an anterior end of the grip (1) and a distal area (3) incorporating an isolated active electrode (31) for the application of radiofrequency current in a body cavity.

These accessories have a cable (4) for connection to a programmable equipment for generating radiofrequency currents (not shown) and provided with means for controlling the current supplied to the active accessory.

The grip (1) of the accessory has a shaped member (11), represented in this case by a plane, appreciable with the touch during the grip of the grip (1) and conveniently arranged to provide an indicative reference of the position of the electrode (31); although the distal area (3) of the accessory is housed in a body cavity and consequently in a non-visible position.

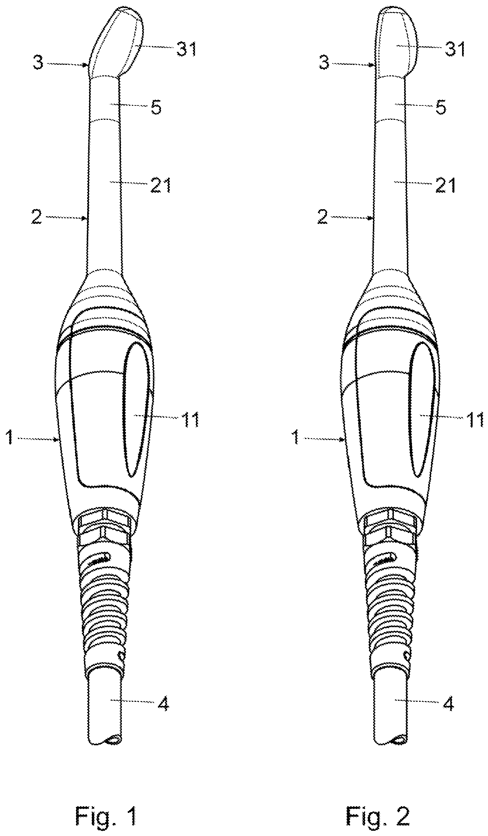

Specifically, in Figure 1, an active intracavitary accessory suitable for applying radio frequency currents in the vagina and rectum has been represented.

In this embodiment the intermediate stem (2) additionally has an electrode (21) in this case cylindrical for the delivery of radiofrequency current in an area of greater amplitude of the body cavity

The electrode (31) of the distal zone (3) is electrically separated from the electrode (21) of the central zone, by means of an insulating member (5).

The electrode (31) can be mounted at an angle forming a variable angle, up to 90°, with the longitudinal axis of the stem (2) as shown in Figure 1, or be aligned with said stem (2) such as shown in the variant embodiment of figure 2.

Both in these embodiments and in those shown below, each of the electrodes (21, 31) included in the active accessory, regardless of its shape, is constituted by a metal electrode isolated by means of a ceramic, plastic or plastic coating. resin, with a thickness between 20 and 500 micrometers.

The active intracavitary accessory of the mentioned figures 1 and 2 can be manufactured in different sizes to adapt its use to different pathologies.

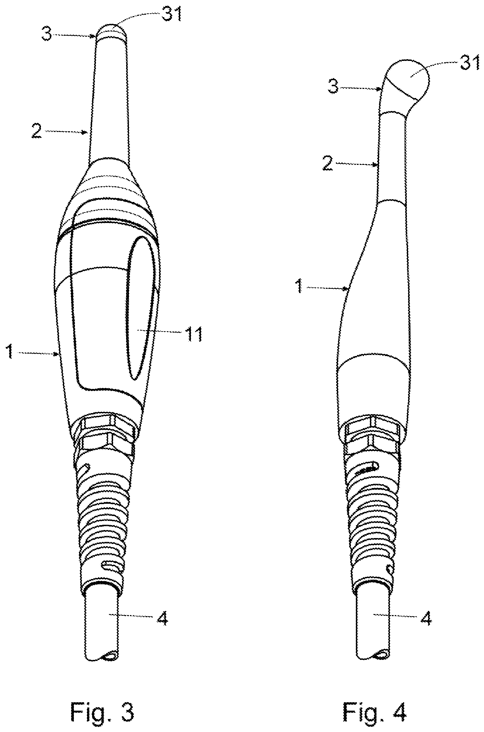

A variant embodiment of the active accessory of the invention is shown in Figure 4 with a configuration suitable for the application of radiofrequency currents in the temporomandibular zone.

In this embodiment, the active intracavitary accessory has a frustoconical stem (2) of insulating material and of adequate length to reach the area to be treated.

The electrode (31) of the distal zone is formed by a metal electrode isolated by a ceramic layer, hemispherical. Frustoconical stem formed by an insulating material, of the appropriate length to reach the area to be treated.

Metal electrode isolated by a hemispherical ceramic layer.

An active accessory suitable for the application of radiofrequency current in soft tissues of the mouth, provided in the distal area of an electrode (31) formed by a metallic electrode isolated by a spherical ceramic layer is shown in figure 4 and mounted at an angle to the axis of the stem (2) which in this case is made of insulating material, the accessory performing the application of radiofrequency currents to the soft tissues of the mouth only by means of the electrode (31) of the distal area (3).

This active accessory has a grip (1) of smaller size than those shown in the previous examples, to optimize the grip for the treatment of soft tissue areas inside the mouth.

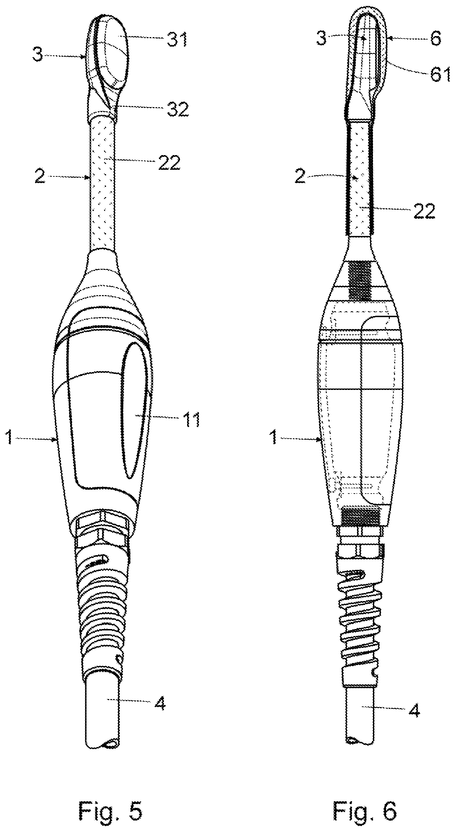

A variant embodiment of the active intracavitary accessory suitable for the application of radiofrequency currents in the gum area is shown in Figure 5.

In this embodiment, the accessory has on the grip (1) of the plane (11) located immediately to indicate the position of the electrode (31) of the distal area (3).

The cylindrical stem (2) of insulating material is provided in this case with a soft coating (22) to protect the teeth during the treatment.

The electrode (31) has a slightly convex shape, and is reinforced at its back (32) with an member of insulating material.

In figure 6, a sheath (6) filled with conductive gel (61) covering the distal area (3) and a part of the stem (2) has been shown, sectioned and mounted on the active accessory of figure 5.

This cover (6) provides a soft texture in order to make the treatment possible in sensitive or hard areas such as the gums, with the goal of facilitating the contact of the electrode with rigid structures of the mouth, also making the treatment more pleasant.

This cover (6) has specific features; specifically: it is made of a biocompatible material with high electrical permittivity; it has an electrically conductive gel filling (61) and has the shape suitable for the correct adaptation thereof on the distal area of any of the active accessories with which it is used.

As mentioned above, to ensure hygiene and prevent infections, the intracavitary elements (intermediate stem and distal area) are used with disposable protective covers, like probe covers, made of biocompatible material, which can be used together with the cover (6) provided with the conductive gel filling, or alone, without the gel cover.

In the invention, an embodiment is also envisaged wherein the stem (2) and the electrode (31) of the distal zone can form an interchangeable member, which is detachable from the grip, and provided with suitable means for its connection and disconnection of said grip.

In this way, said interchangeable member (stem with the distal electrode) is provided with a disposable nature.

Having sufficiently described the nature of the invention, as well as a preferred embodiment, it is stated for the appropriate purposes that the materials, shape, size and arrangement of the elements described may be modified, provided that this does not imply altering the essential features of the invention that are claimed below.

## Claims

1. An active intracavitary accessory, suitable for applying diathermy by radio frequency by means of contact electrodes in the body cavities; comprising: a grip (1), an intermediate stem (2) extending from an front end of the grip (1); a distal zone (3); at least one isolated active electrode (31), arranged in the distal zone (3) for the application of radiofrequency current in a body cavity; a cable (4) for connecting a programmable equipment for generating radiofrequency currents; **characterized in that** the grip (1) of the accessory has a shaped member (11) that can be felt with the touch during the grip of the grip (1) and conveniently arranged to provide an reference indicating the position of the electrode (31), each of the electrodes of the accessory being constituted by an insulated metal electrode by means of a ceramic, plastic or resin coating, with a thickness between 20 and 500 micrometers.

2. The active accessory according to claim 1, suitable for applying radio frequency currents in the vagina and in the rectum **characterized in that** the intermediate stem (2) has an electrode (21) for delivering radiofrequency current.

3. The active accessory according to claim 2, **characterized in that** the electrode (31) of the distal zone (3) is electrically separated from the electrode (21) of the central zone, by means of an insulating member (5).

4. The active accessory according to any one of claims 1 to 3, **characterized in that** the electrode (31) forms a variable angle, up to 90°, with the longitudinal axis of the stem (2).

5. The active accessory according to any one of claims 1 to 3; **characterized in that** the electrode (31) is aligned with the stem (2).

6. The active accessory according to claim 1, suitable for applying radio frequency currents in the temporomandibular zone, **characterized in that** the stem (2) is of insulating material and of adequate length to reach the area to be treated; the electrode (31) of the distal zone being formed by a metal electrode isolated by a hemispherical ceramic layer.

7. The active accessory according to claim 1, suitable for applying radio frequency currents in soft tissues of the mouth, **characterized in that** the electrode (31) of the distal zone is formed by a metal electrode insulated by a spherical shaped ceramic layer and mounted at an angle to the axis of the stem (2).

8. The active accessory according to claim 1, suitable for applying radio frequency currents in the gum area, **characterized in that** the cylindrical stem (2) of insulating material is provided with a soft coating (22) for protect the teeth during the treatment.

9. The active accessory according to claim 8, **characterized in that** the electrode (31) has a slightly convex shape, and is reinforced at its back (32) with a member of insulating material.

10. The active accessory according to any of claims 8 and 9, **characterized in that** it comprises a removable cover (6), filled with a conductive gel (61) of adequate electricity to cover the distal area (3) and a part of the stem (2) of the accessory and that facilitates the contact of the electrode with rigid structures of the mouth.

11. The active accessory according to claim 10, **characterized in that** the cover (6) is made up of a biocompatible material with high electrical permittivity.

12. The active accessory according to any of claims 1 to 10, **characterized in that** it comprises disposable protective covers, type covers probes, of biocompatible material for covering the accessory during use.

13. The active accessory according to claim 1, **characterized in that** it comprises at least one temperature sensor connected to the programmable radio frequency generating equipment in the distal zone.

14. The active accessory according to claim 1, **characterized in that** it is an intelligent accessory and incorporates a fingerprint for its identification individually by the programmable radio frequency generator equipment.

15. The active accessory according to any one of the preceding claims, **characterized in that** the stem (2) and the electrode (31) of the distal zone form an interchangeable member, detachable from the grip, and provided with suitable means for the connection and disconnection thereof from said grip.
